(19) Europäisches Patentamt
European Patent Office
Office européen des brevets

(11) Veröffentlichungsnummer: **0 036 121**
**B1**

(12) **EUROPÄISCHE PATENTSCHRIFT**

(45) Veröffentlichungstag der Patentschrift: **19.10.83**

(51) Int. Cl.³: **C 07 C 13/18,** C 07 C 2/74, C 09 K 5/00, C 10 M 3/10, G 03 C 1/00

(21) Anmeldenummer: **81101478.6**

(22) Anmeldetag: **02.03.81**

(54) **Oligoisobutylcyclohexan, Verfahren zu seiner Herstellung und seine Verwendung.**

(30) Priorität: **14.03.80 DE 3009848**

(43) Veröffentlichungstag der Anmeldung: **23.09.81 Patentblatt 81/38**

(45) Bekanntmachung des Hinweises auf die Patenterteilung: **19.10.83 Patentblatt 83/42**

(84) Benannte Vertragsstaaten: **DE**

(56) Entgegenhaltungen:
**DE-A-2 209 637**
**DE-B-1 643 079**
**DE-B-1 794 018**
**FR-A-2 402 695**
**GB-A-1 438 318**
**US-A-3 925 217**

(73) Patentinhaber: **BAYER AG, *Zentralbereich Patente,* Marken und Lizenzen, D-5090 Leverkusen 1, Bayerwerk (DE)**

(72) Erfinder: **Hentschel, Karl-Heinz, Dr., Heckschenstrasse 89, D-4150 Krefeld (DE)**
Erfinder: **Dhein, Rolf, Dr., Deswatinesstrasse 30, D-4150 Krefeld (DE)**

**EP 0 036 121 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Oligoisobutylcyclohexan, Verfahren zu seiner Herstellung und seine Verwendung

Die vorliegende Erfindung betrifft Oligoisobutylcyclohexan mit einem verzweigten, von Oligoisobuten abgeleiteten Alkylsubstituenten und gegebenenfalls weiteren substituentenverknüpften Cyclohexanring, ein Verfahren zu seiner Herstellung sowie seine Verwendung, beispielsweise als Wärmeübertragungsmedium oder dielektrische Flüssigkeit.

1,1,3,3-Tetramethylbutylsubstituiertes Cyclohexan (Diisobutylcyclohexan) kann auf verschiedenen Wegen synthetisiert werden. Aus J. Amer. Che. Soc. 75, 937 – 9 (1953) ist es bekannt, Phenole im Sinne einer Friedel-Crafts-Alkylierung mit technischem »Diisobuten« zu 1,1,3,3-Tetramethylbutyl-phenolen umzusetzen, zu den entsprechenden Cyclohexanolen katalytisch zu hydrieren, diese mit sauren Katalysatoren bei hohen Temperaturen zu den 1,1,3,3-Tetramethylbutylcyclohexenen zu dehydratisieren und abschließend zu den gewünschten Cyclohexanen zu hydrieren.

In nur 4%iger Ausbeute wurde z. B. 4-Methyl-1-(1,1,3,3-tetramethyl)-butylbenzol durch eine Wurtz-Grignard-Synthese aus p-Tolylmagnesiumbromid und 2-Chlor-2,3,3-trimethylpentan gewonnen und anschließend katalytisch zum 4-Methyl-1-(1,1,3,3-tetramethyl)-butylcyclohexan hydriert [vgl. Zh. Obshchei Khim. 27, 2990 – 3 (Chemical Abstracts 52, 8064 g, 1957)].

Aus Izvest. Vysshikh Ucheb. Zavedenii, Khim. i. Khim. Tekhnol. 4, 657 — 60 (1961) (Chemical Abstracts 56, 7182 c) ist es bekannt, daß die oben genannten beiden Verbindungen in höheren Ausbeuten über eine Friedel-Crafts-Alkylierung von Toluol in Gegenwart von wasserfreiem Aluminiumchlorid und von Nitrobenzol zu erhalten sind.

Aus der FR-AI-2 402 695 ist die Verwendung von Monoalkylcyclohexanen mit 6 bis 14 Kohlenstoffatomen im Alkylrest als Wärmeübertragungsmittel bekannt. Diese Wärmeübertragungsmittel genügen jedoch nicht den in der Anmeldung aufgestellten Forderungen.

Aus der DE-B2-1 794 018 sind Wärmeübertragungsmittel bekannt, die als Hauptbestandteile Alkylbenzole, Hydrinden und Tetralin enthalten. Voraussagen auf andere Wärmeübertragungsmittel sind hieraus jedoch nicht möglich.

Es wurde nun gefunden, daß die bisher nicht bekannten Cyclohexanverbindungen mit einem verzweigten Alkylrest, der sich von höhermolekularem »Oligo-isobuten« wie z. B. Tri- oder Tetraisobuten ableitet, durch Friedel-Crafts-Alkylierung von Aromaten mit trimerem bis decamerem Isobuten und anschließende Hydrierung erhältlich sind.

Gegenstand der Erfindung ist Oligoisobutylcyclohexan der Formel I

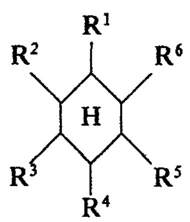

(1)

in der $R^1 - R^5$ gleich oder verschieden sind und Wasserstoff, einen $C_1 - C_4$-Alkyl-, $C_1 - C_4$-Alkoxy- oder $C_1 - C_4$-Alkylthiosubstituenten bedeuten und $R^6$ für einen von trimerem bis decamerem Isobuten abgeleiteten, verzweigten $C_{12} - C_{40}$-Alkylrest steht.

In der obengenannten Formel (1) bedeutet $R^6$ eine verzweigte Alkylgruppe entsprechend einer trimeren bis decameren Isobutens.

Bevorzugt ist Oligoisobutylcyclohexan der Formel 1, in der $R^6$ einen Rest der Formel 2 oder 3 bedeutet

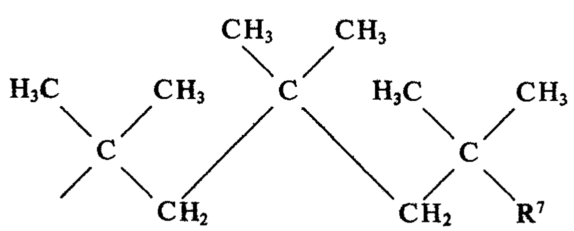

(2)

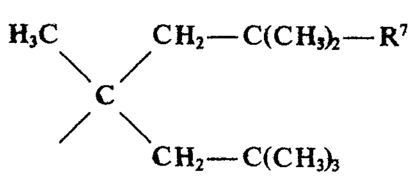

(3)

in denen $R^7$ eine Methylgruppe oder den Rest

$$-CH_2-[C(CH_3)_2-CH_2-]_n-H$$

bedeutet und n eine ganze Zahl von 1–7, vorzugsweise von 1–3 ist.

Besonders bevorzugt ist Oligoisobutylcyclohexan der Formel 4

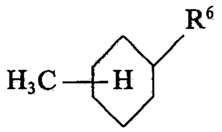

(4)

in der $R^6$ die oben angegebene Bedeutung besitzt.

Die Herstellung der erfindungsgemäßen Verbindungen erfolgt in zwei Stufen. Zunächst wird ein einkerniger, gegebenenfalls substituierter Aromat mit oligomerem Isobuten, z. B. mit technischem Triisobuten in Gegenwart von Friedel-Crafts-Katalysatoren alkyliert und dann das Reaktionsprodukt hydriert.

Zur Alkylierung werden trimeres bis decameres Isobuten oder Gemische dieser Oligoisobutene eingesetzt. Oligoisobuten wie z. B. Triisobutylen oder Tetraisobutylen lassen sich nach bekannten Methoden durch Oligomerisierung von Isobuten in Gegenwart von Friedel-Crafts-Katalysatoren oder Lewis-Säuren herstellen. Geeignete Katalysatoren sind z. B. Schwefelsäure oder saure Tonerden (Chem. Ber. 63 (1930), 104) und $BF_3$/Kieselgur oder $BF_3$/Aluminiumoxid (Dokl. Akad. SSSR 119 (1958), 720, 722, 957, 959). Die erhaltenen Oligoisobuten stellen i. a. Gemische isomerer Olefine dar. So besteht »Triisobutylen« nach J. Amer. Che. Soc. 63 (1941), 2035 hauptsächlich aus 2,2,4,6,6-Pentamethyl-hept-3-en und 2,2,6,6-Tetramethyl-4-methylenheptan sowie aus geringeren Anteilen von 2,4,4,6,6-Pentamethyl-hept-2-en und 2,4,4,6,6-Pentamethyl-hept-1-en.

Zur Alkylierung können sowohl die Isomerengemische von Triisobuten bis zu Decaisobuten eingesetzt werden als auch Gemische dieser homologen Oligoisobutene untereinander. Bevorzugte Oligoisobutene sind Tri-, Tetra-, Penta- oder Hexaisobuten. Besonders bevorzugt ist Triisobuten bzw. dessen Isomerengemische.

Geeignete Aromaten zur Herstellung der erfindungsgemäßen Verbindungen sind solche der Formel 5

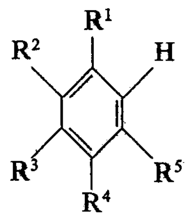

(5)

in der $R^1-R^5$ gleich oder verschieden sind und Wasserstoff, einen $C_1-C_4$-Alkyl-, $C_1-C_4$-Alkoxy oder $C_1-C_4$-Alkylthiosubstituenten bedeuten.

Als Beispiele seien genannt: Benzol und alkylsubstituierte Benzole wie Toluol, o-, m- und p-Xylol, Pentamethylbenzol, Ethylbenzol, Diethylbenzole, Methylethylbenzole, Dimethylbenzole, Cumol, Diisopropylbenzole, Triisopropylbenzole, Cymole, n-Butylbenzol, iso-Butylbenzol, sec.-Butylbenzol und tert.-Butylbenzol, Phenylether und Phenylthioether wie Anisol, Phenetol, Dimethoxybenzole und Phenyl-methylsulfid. Bevorzugt ist Benzol und $C_1-C_4$-monoalkyl-substituiertes Benzol, insbesondere Toluol.

Zur Alkylierung der Aromaten können die üblichen Friedel-Crafts-Katalysatoren verwendet werden, wie z. B. Fluorwasserstoffsäure, Schwefelsäure, Polyphosphorsäure, Phosphorsäure, Aluminiumtrichlorid, Eisen-(III)-chlorid, Antimon-(V)-chlorid, Zinn-(IV)-chlorid, Bortrifluorid, Titantetrachlorid oder Zirkoniumtetrachlorid.

Bevorzugt werden modifizierte Friedel-Crafts-Katalysatoren verwendet, denen zur Verstärkung der Katalysatoraktivität aliphatische oder aromatische Nitroverbindungen wie Nitromethan oder Nitrobenzol zugemischt werden. Besonders bevorzugt ist ein modifizierter Katalysator aus Aluminiumtrichlorid und Nitromethan.

Die Hydrierung der alkylierten Aromaten kann in bekannter Weise erfolgen. Nach dem erfindungsgemäßen Verfahren wird die Hydrierung bevorzugt in Gegenwart eines Hydrierkatalysators wie Nickel (z. B. Raney-Nickel), Platin, Palladium oder Rhenium durchgeführt. Im allgemeinen wird im Temperaturbereich von 150 bis 250°C, bevorzugt von 200 bis 250°C, und im Druckbereich von 150 bis 300 atm hydriert. Der Katalysator wird im allgemeinen in einer Menge von 0,1 bis 10 Gew.-%, bevorzugt von 0,5 bis 5 Gew.-%, bezogen auf das gesamte Reaktionsgemisch, eingesetzt.

Die erfindungsgemäßen Oligoisobutylcyclohexane können als Wärmeübertragungsmedien, dielektrische Flüssigkeiten, z. B. als Transformatorenöle und zur Herstellung photographischer Emulsionen verwendet werden.

Die erfindungsgemäßen Oligoisobutylcyclohexane können außerdem als Schmiermittel für Reibradgetriebe verwendet werden.

Das folgende Beispiel erläutert die Erfindung und beschreibt Herstellung und charakteristische Eigenschaften eines repräsentativen Vertreters der erfindungsgemäßen Verbindungen.

## Beispiel

Durch Einrühren von 266,7 g wasserfreiem Aluminiumtrichlorid in 366 g absolutes Nitromethan stellt man einen modifizierten Friedel-Crafts-Katalysator her, den man in 721,2 g absolutes Toluol einträgt. Unter Rühren wird langsam eine Mischung aus 329,3 g Triisobuten und 180,3 g absolutem Toluol innerhalb von einer Stunde zugetropft und weitere 4 Stunden bei 20–40° C gerührt.

Dann gibt man das Reaktionsgemisch auf 1 l Eiswasser, trennt die organische Phase ab, wäscht diese mit 500 g 10%iger wäßriger Sodalösung und anschließend mit destilliertem Wasser bis zur neutralen Reaktion und trocknet über $CaCl_2$. Danach wird im Wasserstrahlvakuum fraktioniert destilliert.

| | |
|---|---|
| Ausbeute: | 265 g (52% der Theorie, nicht optimiert). |
| Siedepunkt (10 Torr): | 74° C |
| Brechungsindex: | $n_D^{20}$ = 1,4912 |

1280 g des nach dem oben genannten Verfahren hergestellten »Isododecyltoluol« (es handelt sich hier im wesentlichen um das para-Isomere) mit einer vom Triisobuten abgeleiteten »Isododecyl«-Gruppe werden mit 4 Gew.-% eines Nickel-Katalysators zunächst 75 Minuten im Autoklaven unter 150 bis 230 atm Wasserstoffdruck bei einer Temperatursteigerung von 24° auf 205° C, danach 475 Minuten bei 205 bis 235° C und 215 bis 280 atm Wasserstoffdruck und zuletzt 30 Minuten bei 235° C und 275 at Wasserstoffdruck hydriert.

Bei der anschließenden Vakuumdestillation gehen 940 g einer Hauptfraktion bei 60–63° C/10 Torr über (71,8% der Theorie), die folgende Eigenschaft hatte:

Kinemat. Viskositäten:

| | |
|---|---|
| 20° C | 2,27 $mm^2/s$ |
| 37,8° C (100° F) | 1,64 $mm^2/s$ |
| 98,9° C (210° F) | 0,83 $mm^2/s$ |

Stockpunkt: < –70° C.
$n_D^{20}$ = 1,4464.

**Patentansprüche**

1. Oligoisobutylcyclohexan der Formel 1

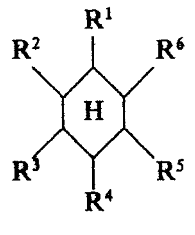

(1)

in der $R^1$–$R^5$ gleich oder verschieden sind und Wasserstoff, einen $C_1$–$C_4$-Alkyl-, $C_1$–$C_4$-Alkoxy- oder $C_1$–$C_4$-Alkylthiosubstituenten bedeuten und $R^6$ für einen von trimerem bis decamerem Isobuten abgeleiteten, verzweigten $C_{12}$–$C_{40}$-Alkylrest steht.

2. Oligoisobutylcyclohexan gemäß Anspruch 1, wobei $R^6$ für einen Rest der Formel 2 oder 3 steht

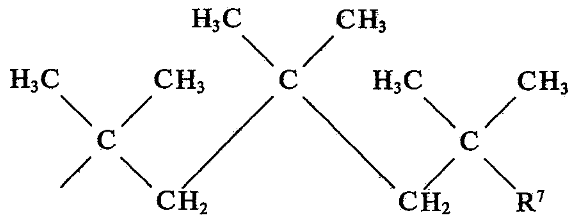

(2)

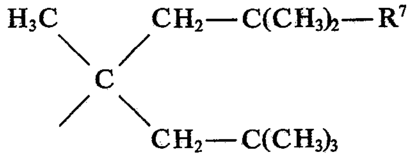

(3)

in denen $R^7$ eine Methylgruppe oder den Rest

$$—CH_2—[C(CH_3)_2—CH_2—]_n—H$$

bedeutet und n eine ganze Zahl von 1–7 ist.

3. Oligoisobutylcyclohexan gemäß Anspruch 2 und Formel 4

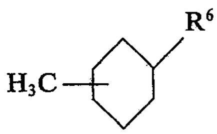

(4)

wobei $R^6$ für einen Rest der Formel 2 oder 3 steht

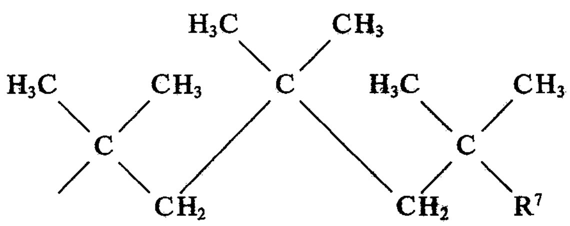

(2)

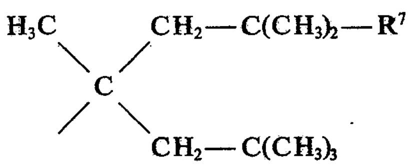

(3)

in denen $R^7$ eine Methylgruppe oder den Rest

$$—CH_2—[C(CH_3)_2—CH_2—]_n—H$$

bedeutet und n eine ganze Zahl von 1–3 ist.

4. Verfahren zur Herstellung von Oligoisobutylcyclohexan gemäß Anspruch 1, 2 oder 3, dadurch gekennzeichnet, daß eine aromatische Verbindung der Formel 5

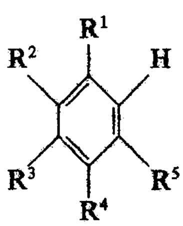

(5)

in der $R^1 - R^5$ gleich oder verschieden sind und Wasserstoff, einen $C_1 - C_4$-Alkyl-, $C_1 - C_4$-Alkoxy- oder $C_1 - C_4$-Alkylthiosubstituenten bedeuten mit trimerem bis decamerem Isobuten oder deren Gemischen in Gegenwart eines Friedel-Crafts-Katalysators alkyliert und anschließend hydriert wird.

5. Verfahren nach Anspruch 4, dadurch gekennzeichnet, daß ein mit einer aromatischen oder aliphatischen Nitroverbindung modifizierter Friedel-Crafts-Katalysator verwendet wird.

6. Verfahren nach Anspruch 5, dadurch gekennzeichnet, daß als Friedel-Crafts-Katalysator Aluminiumtrichlorid und als aliphatische Nitroverbindung Nitromethan verwendet wird.

7. Verfahren nach Anspruch 6, dadurch gekennzeichnet, daß Toluol mit Triisobuten oder dessen Isomerengemisch alkyliert wird.

8. Verwendung von Oligoisobutylcyclohexan gemäß Anspruch 1, 2 oder 3 als Wärmeübertragungsmedium, dielektrische Flüssigkeit oder zur Herstellung photographischer Emulsionen.

9. Verwendung von Oligoisobutylcyclohexan gemäß Anspruch 1, 2 oder 3 als Schmiermittel für Reibradgetriebe.

**Claims**

1. Oligoisobutylcyclohexane of the formula 1

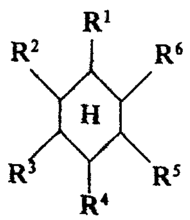

(1)

wherein $R^1$—$R^5$ are identical or different and denote hydrogen or a $C_1$—$C_4$-alkyl, $C_1$—$C_4$-alkoxy or $C_1$— $C_4$-alkylthio substituent and $R^6$ denotes a branched chain $C_{12}$—$C_{40}$-alkyl group derived from trimeric to decameric isobutene.

2. Oligoisobutylcyclohexane according to claim 1, wherein $R^6$ denotes a group of Formula 2 oder 3

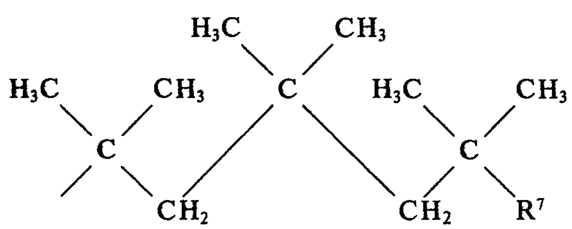

(2)

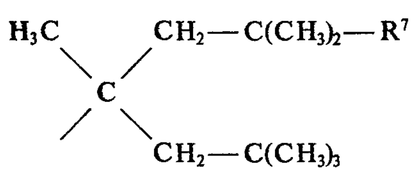

(3)

wherein $R^7$ denotes a methyl group or the group

$$—CH_2—[C(CH_3)_2—CH_2—]_n—H$$

and n represents an integer of from 1 to 7.

3. Oligoisobutylcyclohexane according to claim 2 and formula 4

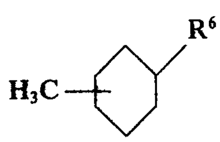

(4)

wherein $R^6$ denotes a group of Formula 2 or 3

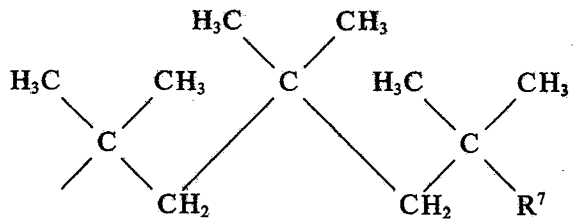

(2)

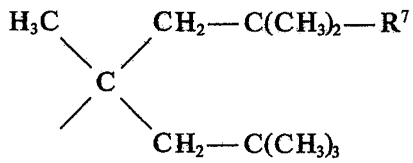

(3)

wherein $R^7$ denotes a methyl group or the group

$$-CH_2-[C(CH_3)_2-CH_2-]_n-H$$

and n represents an integer of from 1 to 3.

4. A process for the preparation of oligoisobutylcyclohexane according to claim 1, 2 or 3, characterised in that an aromatic compound of the formula 5

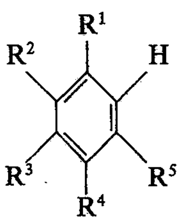

(5)

wherein $R^1-R^5$ are identical or different and denote hydrogen, a $C_1-C_4$-alkyl substituent, a $C_1-C_4$-alkoxy substituent or a $C_1-C_4$-alkylthio substituent, is alkylated with trimeric to decameric isobutene in the presence of a Friedel-Crafts catalyst and subsequently hydrogenated.

5. A process according to claim 4, characterised in that a Friedel-Crafts catalyst modified with an aromatic or aliphatic nitro compound is used.

6. A process according to claim 5, characterised in that the Friedel-Crafts catalyst used is aluminium trichloride and the aliphatic nitro compound used is nitromethane.

7. A process according to claim 6, characterised in that toluene is alkylated with triisobutene or its isomeric mixture.

8. The use of oligoisobutylcyclohexane according to claim 1, 2 or 3 as a heat transfer medium or a dielectric liquid or for the preparation of photographic emulsions.

9. The use of oligoisobutylcyclohexane according to claim 1, 2 or 3 as a lubricant for friction drives.

**Revendications**

1. Oligoisobutylcyclohexane de formule 1

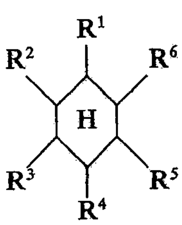

(1)

dans laquelle $R^1-R^5$ sont identiques ou différents et représentent l'hydrogène, un substituant en $C_1-C_4$, alcoxy en $C_1-C_4$ ou alkylthio en $C_1-C_4$ et $R^6$ représente un reste alkyle en $C_{12}-C_{40}$ ramifié, dérivé de l'isobutène trimère à décamère.

2. Oligoisobutylcyclohexane selon la revendication 1, caractérisé en ce que $R^6$ est un reste de formule 2 ou 3

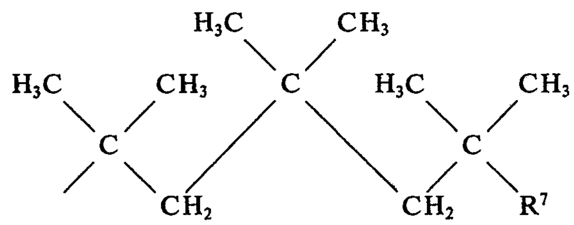

(2)

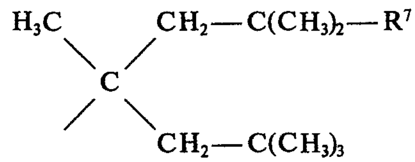

(3)

dans lesquels $R^7$ représente un groupe méthyle ou le reste

$$—CH_2—[C(CH_3)_2—CH_2—]_n—H$$

et n est un nombre entier de 1 à 7.

3. Oligoisobutylcyclohexane selon la revendication 2 et la formule 4

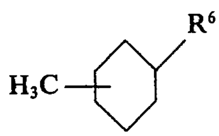

(4)

dans laquelle $R^6$ représente un reste de formule 2 ou 3

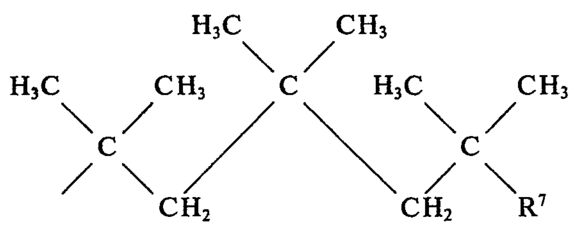

(2)

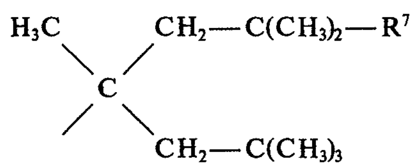

(3)

dans laquelles $R^7$ représente un groupe méthyle ou le reste

$$—CH_2—[C(CH_3)_2—CH_2—]_n—H$$

et n est un nombre entier de 1 à 3.

4. Procédé pour la fabrication d'oligoisobutylcyclohexane selon la revendication 1, 2 ou 3, caractérisé en ce qu'un composé aromatique de formule 5

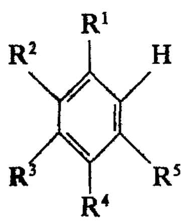

(5)

dans laquelle $R^1 - R^5$ sont identiques ou différents et représentent l'hydrogène, un substituant alkyle en $C_1 - C_4$, alcoxy en $C_1 - C_4$ ou alkylthio en $C_1 - C_4$ est alkylé avec l'isobutène trimère à décamère ou leurs mélanges en présence d'un catalyseur Friedel-Crafts et ensuite hydrogéné.

5. Procédé selon la revendication 4, caractérisé en ce que l'on utilise un catalyseur Friedel-Crafts modifié par un composé nitro aromatique ou aliphatique.

6. Procédé selon la revendication 5, caractérisé en ce que l'on utilise comme catalyseur Friedel-Crafts le trichlorure d'aluminium et comme composé nitro aliphatique le nitrométhane.

7. Procédé selon la revendication 6, caractérisé en ce que le toluène est alkyle avec le triisobutène ou un mélange de ses isomères.

8. Utilisation de l'oligoisobutylcyclohexane selon la revendication 1, 2 ou 3, comme milieu caloporteur, liquide diélectrique ou pour la fabrication d'émulsions photographiques.

9. Utilisation de l'oligoisobutylcyclohexane selon la revendication 1, 2 ou 3, comme lubrifiant pour engrenage à roues à friction.